# EUROPÄISCHE PATENTSCHRIFT

(11) **EP 0 707 008 B1**
(45) Veröffentlichungstag und Bekanntmachung des Hinweises auf die Patenterteilung: **16.01.2002**
(21) Anmeldenummer: 95115743.7
(22) Anmeldetag: 06.10.1995
(51) Int. Cl.: C07F 1/02, C07C 45/68

(54) **Verfahren zur Herstellung eines Monolithium-acetylid-Ammoniak-Komplexes**
Process for the preparation of a monolithium-acetylide-ammonia complex
Procédé de préparation de complexes de monoacétylures de lithium et ammoniac

(30) Priorität: 13.10.1994 DE 4436498
(43) Veröffentlichungstag der Anmeldung: 17.04.1996
(73) Patentinhaber: BASF AKTIENGESELLSCHAFT, 67056 Ludwigshafen (DE)
(72) Erfinder: Rühl, Thomas, Dr., D-67227 Frankenthal (DE); Müller, Rolf, D-68766 Hockenheim (DE); Henkelmann, Jochem, Dr., D-68165 Mannheim (DE); Heider, Marc, Dr., D-67433 Neustadt (DE)

(56) Entgegenhaltungen:
- CH-A- 642 936
- US-A- 3 557 220

## Beschreibung

Die vorliegende Erfindung betrifft ein verbessertes Verfahren zur Herstellung eines Monolithiumacetylid-Ammoniak-Komplexes durch Umsetzung von Lithiumamid und Acetylen in Ammoniak. Weiterhin betrifft sie die Verwendung des so hergestellten Acetylidkomplexes zur Ethinylierung α,β-ungesättigter Ketone und Aldehyde.

Die CH-A 642 936 beschreibt die Herstellung eines Monolithiumacetylid-Ammoniak-Komplexes aus Lithiumamid und Acetylen. Lithiumamid wird dazu in einem inerten organischen Lösungsmittel suspendiert und bei unter 30°C, bevorzugt unter 10°C, mit Acetylen versetzt. Diese Herstellweise kann nicht völlig befriedigen, da sich in den organischen Lösungsmitteln Dilithiumacetylid (Lithiumcarbid) bildet, das unreaktiv ist und aus der Lösung ausfällt. Weiterhin sind die Reaktionszeiten relativ lang. Das Lithiumamid reagiert unter den genannten Bedingungen außerdem nicht vollständig ab, was in Folgereaktionen mit α,β-ungesättigten Carbonylverbindungen zu deren unerwünschter Polymerisation führen kann.

Aus Chimia 40 (1986) 323 ist bekannt, daß der Monolithiumacetylid-Ammoniak-Komplex thermisch nicht stabil ist und in Lithiumcarbid, Acetylen und Ammoniak zerfällt.

Es bestand die Aufgabe, ein Verfahren bereitzustellen, daß die genannten Nachteile bekannter Verfahren vermeidet.

Demgemäß wurde das eingangs definierte Verfahren gefunden, das dadurch gekennzeichnet ist, daß man die Umsetzung bei -10°C bis 30°C vornimmt.

Beim erfindungsgemäßen Verfahrensprodukt handelt es sich wahrscheinlich um den Komplex LiC≡CH·NH₃.

Erfindungsgemäß wird Lithiumamid mit Acetylen umgesetzt. Lithiumamid ist als Handelsprodukt erhältlich oder kann beispielsweise durch Umsetzung von Lithiumhydrid mit Ammoniak unter Wasserstoffabspaltung hergestellt werden. Das erfindungsgemäß zu verwendende Acetylen ist bevorzugt acetonfrei, um unerwünschte Nebenreaktionen zu verhindern. Es kann mit inerten Gasen wie Stickstoff oder Argon verdünnt werden; bevorzugt wird es aber unverdünnt eingesetzt.

Im allgemeinen können 1 bis 10 mol Acetylen pro Mol Lithiumamid verwendet werden, bevorzugt sind jedoch 1 bis 3 mol.

Das erfindungsgemäße Verfahren wird in Ammoniak als Lösungsmittel ausgeführt. In der Regel werden 7 bis 30 mol Ammoniak pro Mol Lithiumamid verwendet.

In einer bevorzugten Ausführungsform wird zunächst Lithiumamid mit Ammoniak versetzt. Dann wird unter Rühren Acetylen zum Reaktionsansatz gegeben.

Da sich das Acetylen relativ schnell im Ammoniak löst und abreagiert, kann es so zugegeben werden, daß der Druck während der Acetylen-Zugabe praktisch konstant bleibt. Es hat sich als vorteilhaft erwiesen, die Reaktion unter dem Eigendruck des Ammoniaks bei der gewählten Reaktionstemperatur vorzunehmen, was je nach Reaktionstemperatur einem Druck von ca. 3 bis 15 bar entspricht.

Die Reaktionstemperatur beträgt -10 bis 30°C, bevorzugt 0 bis 20°C.

Das erfindungsgemäße Verfahren kann in Rührkesseln oder Druckbehältern ausgeübt werden.

Die Reaktion zum Monolithiumacetylid-Ammoniak-Komplex ist in der Regel nach wenigen Minuten beendet. Es hat sich als zweckmäßig erwiesen, solche Lösungen des Acetylidkomplexes herzustellen, die ca. 1- bis 3-molar am Verfahrensprodukt sind.

Das erfindungsgemäße Verfahren erlaubt eine praktisch quantitative Acetylenumsetzung, wobei sich Lithiumcarbid als Nebenprodukt nicht nachweisen läßt. Es ist technisch äußerst wünschenswert, die Reaktion bei den genannten Temperaturen ablaufen zu lassen, da dazu keine teueren Kälteaggregate erforderlich sind. Wenn bei Temperaturen unter Raumtemperatur gearbeitet werden soll, können diese Temperaturen mit Kältesolen, wie sie in chemischen Betrieben üblicherweise vorhanden sind, in einfacher Weise eingestellt werden.

Der erfindungsgemäß hergestellte Monolithiumacetylid-Ammoniak-Komplex kann in an sich bekannter Weise weiter umgesetzt werden. Die den Komplex enthaltene Ammoniaklösung kann unter Verdampfen des Ammoniaks entspannt werden. Der so erhaltene Komplex kann dann in einem inerten organischen Lösungsmittel wie Toluol, Tetrahydrofuran oder Methyl-tert.-butylether aufgenommen werden und mit α,β-ungesättigten Aldehyden oder Ketonen wie Methylvinylketon oder Aceton unter Addition der Acetylidgruppe umgesetzt werden. Nach hydrolytischer Aufarbeitung erhält man so wertvolle Zwischenprodukte für Carotinoidsynthesen (s. CH-A 642 936).

### Beispiele

### Beispiel 1

In einen 150 l Autoklaven (Nenndruck 25 bar) wurden zunächst 8 kg (348 mol) Lithiumamid und dann 100 l flüssiger Ammoniak gegeben. Es stellte sich ein Druck von ca. 8 bar ein.

Unter Rühren wurden 8 m³ (357 mol) Acetylen in den Ansatz gepreßt. Nach 15 Minuten wurde der Autoklav entspannt und der entstandene Monolithiumacetylid-Ammoniak-Komplex in 100 l Methyl-tert.-butylether suspendiert. Lithiumcarbid war nicht nachweisbar.

Bei -5°C wurden danach 22,5 kg Methylvinylketon in die Suspension gegeben. Nach Hydrolyse und Destillation erhielt man 3-Hydroxy-3-methyl-penten-4-in-1 in 87 % Ausbeute.

### Beispiel 2

Analog zu Beispiel 1 wurde die Synthese des Monolithiumacetylid-Ammoniak-Komplexes bei 0°C und einem Eigendruck von 2,5 bar durchgeführt.

Die nachfolgende Umsetzung mit Methylvinylketon ergab 89 % Ausbeute.

### Beispiel 3

Der Monolithiumacetylid-Ammoniak-Komplex wurde analog zu Beispiel 1 hergestellt.

Die nachfolgende Umsetzung mit 20,3 kg (350 mol) Aceton ergab 98 % Methylbutinol.

### Beispiel 4

Der Monolithiumacetylid-Ammoniak-Komplex wurde analog zu Beispiel 1 hergestellt.

Die nachfolgende Umsetzung mit 70,7 kg (350 mol) β-Ionon ergab 94 % ethinyliertes Ionon.

## Patentansprüche

1. Verfahren zur Herstellung eines Monolithiumacetylid-Ammoniak-Komplexes durch Umsetzung von Lithiumamid und Acetylen in Ammoniak, **dadurch gekennzeichnet, daß** man die Umsetzung bei einer Temperatur von -10 bis 30°C vornimmt.

2. Verfahren nach Anspruch 1, **dadurch gekennzeichnet, daß** man die Umsetzung unter dem Eigendruck des Ammoniaks vornimmt.

3. Verwendung eines nach einem Verfahren gemäß Anspruch 1 hergestellten Monolithiumacetylid-Ammoniak-Komplexes zur Ethinylierung von α,β-ungesättigten Ketonen und Aldehyden.

## Claims

1. A process for the preparation of a monolithium acetylide/ammonia complex which comprises reacting lithium amide and acetylene in ammonia at a temperature of from -10 to 30°C.

2. A process as defined in claim 1, wherein the reaction is carried out under the autogeneous pressure of the ammonia.

3. The use of a monolithiumacetylide/ammonia complex prepared by a process as defined in claim 1 in order to ethynylate α,β-unsaturated ketones and aldehydes.

## Revendications

1. Procédé pour la préparation d'un complexe acétylure monolithique-ammoniac par réaction de l'amidure de lithium et de l'acétylène dans l'ammoniac, **caractérisé par le fait que** la réaction est réalisée à une température de -10 à +30° C.

2. Procédé selon la revendication 1, **caractérisé par le fait que** la réaction est réalisée sous la pression propre de l'ammoniac.

3. Utilisation d'un complexe acétylure monolithique-ammoniac préparé par un procédé selon la revendication 1 pour l'éthynylation de cétones et d'aldéhydes alpha, bêta-insaturés.
